(19) Europäisches Patentamt
European Patent Office
Office européen des brevets

(11) **EP 4 134 135 A1**

(12) **DEMANDE DE BREVET EUROPEEN**

(43) Date de publication:
**15.02.2023  Bulletin 2023/07**

(21) Numéro de dépôt: **22177302.1**

(22) Date de dépôt: **03.06.2022**

(51) Classification Internationale des Brevets (IPC):
**A61Q 19/02** [(2006.01)]    **A61Q 19/08** [(2006.01)]
**A61K 8/9789** [(2017.01)]

(52) Classification Coopérative des Brevets (CPC):
**A61Q 19/08; A61K 8/9789; A61Q 19/02**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité:  **07.06.2021  FR 2105956**

(71) Demandeur: **Lyster**
**86360 Chasseneuil-du-Poitou (FR)**

(72) Inventeurs:
• **BOURGETEAU, Vincent**
  **69007 LYON (FR)**
• **PIGNOLET, François**
  **69007 LYON (FR)**

(74) Mandataire: **Opilex**
**32, rue Victor Lagrange**
**69007 Lyon (FR)**

(54) **HYDROLYSAT DE TOURTEAU DE COLZA, PROCÉDÉ DE PRÉPARATION ET UTILISATION EN ALIMENTAIRE ET EN COSMÉTIQUE NOTAMMENT POUR TRAITER LE VIEILLISSEMENT CUTANÉ ET DÉPIGMENTER LA PEAU**

(57)    [Hydrolysat de tourteau de colza comprenant de 0,3 à 3% en poids de protéines de poids moléculaire inférieur à 30 kDa et de 0,01 % à 0,5 % en poids de composés volatils, dont 0,01 à 0,1% en poids de composés volatils azotés par rapport au poids total de l'hydrolysat, procédé de préparation, compositions alimentaires et cosmétiques et utilisations en cosmétique en particulier en tant qu'agent pour prévenir et/ou traiter les signes du vieillissement cutané et pour blanchir, éclaircir et/ou dépigmenter la peau.

[Fig. 1]

EP 4 134 135 A1

**Description**

La présente invention se rapporte au domaine de la valorisation du tourteau de colza. Plus spécifiquement l'invention concerne un extrait de tourteau de colza hydrolysé ainsi que son utilisation en alimentaire et en cosmétique notamment en tant qu'agent pour prévenir et/ou traiter les signes du vieillissement cutané et pour blanchir, éclaircir et/ou dépigmenter la peau.

La peau est la principale barrière de protection du corps humain à l'égard des agressions extérieures telles que la pollution atmosphérique, les variations climatiques et le rayonnement UV.

La peau est constituée de trois couches : l'épiderme, le derme et l'hypoderme. L'épiderme, qui est en contact avec l'extérieur, est plus particulièrement concerné par les agressions extérieures. L'épiderme est recouvert par un film hydrolipidique et est composé de quatre couches : la couche cornée, la couche granuleuse, la couche épineuse et la couche basale. La peau, et en particulier l'épiderme, est ainsi constamment soumis à des stress qui engendrent son vieillissement.

On distingue généralement le vieillissement intrinsèque et le vieillissement extrinsèque.

Le vieillissement intrinsèque, encore appelé vieillissement physiologique provoque un ralentissement du renouvellement des kératinocytes qui sont les cellules des couches superficielles la peau, en particulier de l'épiderme. La réduction du tissus adipeux sous-cutané et l'apparition de fines rides sont les principales manifestations du vieillissement intrinsèque.

Le vieillissement extrinsèque se caractérise par une perte d'élasticité de la peau, une altération de la fonction barrière de la peau et l'apparition de rides profondes. Le vieillissement extrinsèque est essentiellement dû aux expositions répétées de la peau au soleil, il est également associé à l'apparition de taches plus foncées qui génèrent des zones d'aspect non-homogène. Ce vieillissement est également nommé photo-vieillissement ou vieillissement actinique.

Ces taches foncées sont généralement dues à une concentration importante de mélanine, qui est le pigment responsable de la coloration de la peau, dans les mélanocytes situés à la surface de la peau.

Le mécanisme de formation de la pigmentation de la peau, c'est-à-dire de la mélanine, est particulièrement complexe et fait intervenir schématiquement les principales étapes suivantes, au niveau des mélanocytes de la couche basale :

**Tyrosine→DOPA→Dopaquinone →leucodopachrome → dopachrome → mélanine**

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase.

Il demeure par conséquent toujours un besoin de nouvelles compositions cosmétiques destinées à être appliquées sur la peau et qui présentent des propriétés anti-âge et dépigmentantes.

Les inventeurs ont montré qu'une solution à ce problème technique pouvait être apportée au moyen d'un extrait particulier de tourteau de colza.

Le colza est une plante annuelle à fleurs jaunes de la famille des Brassicacées. Le colza est essentiellement cultivé pour l'alimentation animale et la production d'huile alimentaire. Depuis quelques décennies, le colza est également utilisé, en quantité croissante, en tant que biocarburant.

L'huile de colza est obtenue par pressage, puis raffinage plus ou moins poussé du colza. La production d'huile de colza génère une grande quantité de sous-produits solides appelés tourteaux de colza.

Le tourteau de colza constitue une source de protéines et, à ce titre, il est utilisé dans des formulations pour l'alimentation animale plus particulièrement pour les bovins, porcins et ovins.

Il demeure cependant toujours un besoin de trouver de nouvelles voies de valorisation du tourteau de colza à haute valeur ajoutée.

De manière surprenante et avantageuse les inventeurs ont mis en évidence un extrait particulier de tourteau de colza obtenu par un procédé d'hydrolyse alcaline particulièrement innovant dans le traitement des tourteaux de colza, et présentant des effets avantageux en alimentaire et en cosmétique en application sur la peau, plus particulièrement des propriétés cosmétiques notamment anti-âge et dépigmentantes.

La présente invention a pour objet un hydrolysat de tourteau de colza comprenant de 0,3 à 3% en poids de protéines de poids moléculaire inférieur à 30 kDa par rapport au poids total de l'hydrolysat et de 0,01 % et 0,5 % en poids de composés volatils par rapport au poids total de l'hydrolysat dont 0,01 % à 0,1% en poids de composés volatils azotés par rapport au poids total de l'hydrolysat.

Dans le présent texte, l'hydrolysat est également nommé extrait de tourteau de colza ou extrait de tourteau de colza hydrolysé.

Plus particulièrement, l'hydrolysat selon l'invention est tel que la phase des composés volatils, encore appelée phase volatile, comprend de 25% à 35% de composés azotés en poids par rapport au poids total de la phase des composés volatils, de 30% à 45% de composés aromatiques non azotés en poids par rapport au poids total de la phase des composés volatils. En particulier la phase des composés aromatiques non azotés comprennent des composés d'huiles essentielles.

La présente invention a aussi pour objet un procédé de préparation de l'hydrolysat selon l'invention comprenant au moins les étapes suivantes, dans cet ordre :

- fournir des graines de colza ;
- presser les graines de colza et récupérer le tourteau ;
- placer le tourteau de colza en phase aqueuse ;
- soumettre le tourteau à au moins une étape d'hydrolyse au moyen d'une solution alcaline à un pH allant de 13 à 14 pendant une durée allant de 30 minutes à 4 heures,
- ajouter un acide faible de manière à ajuster le pH à une valeur allant de 4 à 5 ;
- effectuer une séparation solide/ liquide,
- récupérer l'hydrolysat obtenu.

Avantageusement, lors de l'hydrolyse, le ratio masse de tourteau de colza / masse de la solution alcaline est compris entre 1/12 et 1/8 et de manière préférée d'environ 1/10.

La présente invention vise encore une composition cosmétique ou alimentaire comprenant au moins un hydrolysat selon la présente invention ou préparé suivant le procédé selon la présente invention, de préférence en une teneur allant de 0,001 à 30 % en poids et de préférence de 0,1 à 20 % en poids par rapport au poids total de la composition.

L'hydrolysat selon l'invention permet de fournir un produit renfermant deux types d'actifs d'intérêts en cosmétique c'est-à-dire à la fois des composés volatils notamment azotés et des protéines de bas poids moléculaire.

Les protéines présentes dans l'hydrolysat selon l'invention ont un poids moléculaire faible par rapport au poids moléculaire des protéines, majoritairement des cruciférines, présentes dans le colza à l'état natif qui sont de l'ordre de 300 kDa.

Ainsi l'hydrolysat de tourteau de colza et la composition cosmétique selon l'invention permettent avantageusement de prévenir et/ou diminuer les signes de vieillissement cutané, en particulier la perte d'élasticité de la peau, l'altération de la fonction barrière de la peau l'apparition de rides et/ou de ridules. Il a été observé que les peaux traitées sont plus fermes.

L'hydrolysat de tourteau de colza et la composition cosmétique selon l'invention permettent également de blanchir et/ou éclaircir et/ou dépigmenter la peau du visage et/ou du corps de manière durable. Ils permettent aussi de réduire la taille des taches brunes pigmentaires et donc de rendre plus homogènes des zones précises du visage et/ou du corps. Il a été observé que les peaux traitées sont moins ternes, plus homogènes, moins sèches. Les peaux traitées sont plus lumineuses, plus rayonnantes.

Ces compositions cosmétiques sont en outre stables, non irritantes, non toxiques, non allergisantes pour la peau.

D'autres aspects, avantages, propriétés de la présente invention sont présentés dans la description et les exemples qui suivent.

**Définitions**

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Un intervalle défini comme étant « compris entre la valeur A et la valeur B » inclut les valeurs A et B et est donc équivalent à un intervalle « allant de la valeur A à la valeur B ». En outre, l'expression « au moins » comprend la valeur énoncée après, par exemple, « au moins 5 % » doit être compris comme comprenant également « 5 % ». L'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence. De préférence, ces valeurs sont destinées à comprendre des variations de $\pm$ 5 %.

Dans le présent texte, la forme au singulier d'un mot comprend le pluriel et inversement, sauf si le contexte indique clairement le contraire. Ainsi, les références « un », « une » et « le/la » comprennent généralement les pluriels des termes respectifs. Par exemple, une référence à un « procédé » ou une « composition » comprend une pluralité de ces « procédés » ou « compositions». En particulier, dans le présent texte, les termes « tourteau de colza » et « tourteaux de colza » sont utilisés de manière indifférentiée.

Par « peau » on entend l'épiderme du visage ou du corps et en particulier du visage, du cou, du décolleté et des mains.

Les expressions « blanchiment », « éclaircissement » et « dépigmentation » utilisées pour définir les propriétés des compositions selon l'invention ont la même signification, de même que les expressions « blanchir », « éclaircir » et « dépigmenter ».

Par « prévenir » ou « prévention » on entend le fait de diminuer, au moins en partie, le risque de survenue d'un effet.

Les compositions selon la présente invention sont des compositions cosmétiques, non thérapeutiques. Ces compositions sont destinées à être appliquées sur les peaux saines, par simple massage pour traiter l'épiderme. Elles sont conformes au règlement CE 1223/2009.

## Hydrolysat

L'hydrolysat selon la présente invention est préparé à partir de tourteaux de colza, plus particulièrement à partir de graines de colza (*Brassica napus*).

Il est connu que les graines de *Brassica napus* sont utilisées pour l'extraction de leur huile, utile pour l'autoconsommation ou en soins médicinaux traditionnels divers. Le tourteau obtenu après avoir déshuilé la graine est un déchet actuellement utilisé pour la nourriture notamment des animaux.

Selon un mode de réalisation préférentiel, l'hydrolysat selon l'invention est obtenu à partir du tourteau des graines non décortiquées de *Brassica napus.*

Selon la présente invention, les tourteaux de colza sont récupérés après l'extraction de l'huile de colza par pressage puis hydrolysés.

L'hydrolysat selon l'invention comprend de 0,3 à 3% en poids de protéines de poids moléculaire inférieur à 30 kDa, par rapport au poids total de l'hydrolysat et de 0,01 % à 0,5 % en poids de composés volatils par rapport au poids total de l'hydrolysat dont 0,01 à 0,1% en poids de composés volatils azotés par rapport au poids total de l'hydrolysat, l'hydrolysat considéré étant sous forme liquide.

Plus particulièrement, l'hydrolysat comprend environ 1,5% en poids de protéines de poids moléculaire inférieur à 30 kDa, par rapport au poids total de l'hydrolysat. La teneur en protéines est mesurée selon la méthode de mesure de la densité optique de Lowry à 750 nm.

Cette méthode bien connue de l'homme du métier est notamment décrite dans « Protein measurement with the Folin phenol reagent », Oliver H. Lowry, Nira J. Rosebrough, A Lewis Farr et Rose J. Randall, J. biol. Chem., vol. 193, no 1, 1951, p. 265-275.

En outre lesdites protéines de poids moléculaire inférieur à 30 kDa sont solubles, notamment dans l'eau à température ambiante, puisqu'elles sont présentes dans l'hydrolysat sous forme liquide c'est-à-dire dans le filtrat obtenu par le procédé selon l'invention.

Plus particulièrement, l'hydrolysat de tourteau de Colza selon l'invention présente trois bandes protéiques caractéristiques de faible poids moléculaire :

- la première, nommée P1, de taille apparente d'environ 17kDa ;
- la deuxième, nommée P2, de taille apparente comprise entre 17 et 20kDa,
- la troisième, nommée P3, de taille apparente d'environ 25kDa.

L'hydrolysat selon la présente invention se distingue par un taux de composés volatils allant de 0,01 % à 0,5 % en poids par rapport au poids total de l'hydrolysat, dont 0,01 à 0,1% en poids de composés volatils azotés par rapport au poids total de l'hydrolysat.

Par « composé volatil » au sens de la présente invention, on entend un composé dont la pression de vapeur à 20°C (293,15 K) est supérieure ou égale à 0,01 kPa. Cette définition est en conformité avec la directive du Conseil Européen 1999/13/CE.

Par « composé azoté » au sens de la présente invention, on entend un composé comprenant au moins un atome d'azote.

L'hydrolysat de tourteau de colza selon l'invention est obtenu sous la forme d'un hydrolysat liquide présentant une densité, par rapport à l'eau, supérieure à 1 et préférentiellement autour de 1,1.

L'hydrolysat de tourteau de colza selon l'invention comprend un taux de matières sèches compris entre 10 et 15 %, préférentiellement environ 12.5% en poids par rapport au poids total de l'hydrolysat.

**Procédé de préparation de l'hydrolysat**

L'hydrolysat de tourteau de colza selon la présente invention est obtenu par hydrolyse au moyen d'une solution alcaline à un pH allant de 13 à 14, de préférence obtenu au moyen d'une base forte, de manière préférée choisie parmi NaOH, KOH et CaOH.

Comme déjà mentionnné, le procédé de préparation de l'hydrolysat de tourteau de colza comprend au moins les étapes suivantes, dans cet ordre :

- fournir des graines de colza ;
- presser les graines de colza et récupérer le tourteau;
- placer le tourteau de colza en phase aqueuse ;
- soumettre le tourteau à au moins une étape d'hydrolyse au moyen d'une solution alcaline à un pH allant de 13 à 14 pendant une durée allant de 30 minutes à 4 heures,
- ajouter un acide faible de manière à ajuster le pH à une valeur allant de 4 à 5 ;
- effectuer une séparation solide/ liquide ;
- récupérer l'hydrolysat obtenu.

Généralement, les graines de colza obtenues destinées à être utilisées dans le procédé selon l'invention sont non décortiquées.

De préférence elles sont séchées ou laissées sécher de manière à obtenir un taux d'humidité inférieur à 7%.

Puis les graines sont pressées de manière à séparer l'huile du reste de la graine et à obtenir le tourteau. Généralement le tourteau comprend moins de 5% en poids d'huile dite « huile résiduelle ».

Selon une variante préférée, le tourteau est ensuite broyé mécaniquement avec tout type de broyeurs mécaniques tels que des systèmes à marteaux, à fléau, à couteaux, à concassage/déchiquetage, à billes ou boulets ou à pilon, mais aussi avec tout type de cryobroyeur.

Le broyage du tourteau de colza permet avantageusement d'obtenir la taille de particule appropriée. Par « taille », on entend la longueur de la plus grande dimension des particules. Généralement, les particules de tourteau de colza présentent une taille maximale proche de 5 mm, de préférence proche de 3 mm, et de manière préférée comprise entre 1 et 2 mm.

Le tourteau, avantageusement broyé, est ensuite mis en dispersion en phase aqueuse. Avantageusement, la phase aqueuse ne comprend pas de polyvinylpyrrolidone.

Avantageusement, le tourteau de colza n'est pas soumis à une extraction à l'éthanol avant l'hydrolyse alcaline.

Puis une étape d'hydrolyse alcaline de préférence avec au moins une base forte est réalisée. La base forte est généralement choisie parmi l'hydroxyde de sodium (NaOH) l'hydroxyde de potassium (KOH) ou et l'hydroxyde de calcium (CaOH) ; de préférence l'hydroxyde de sodium. Avantageusement la solution de base forte présente une molarité comprise entre 0,8 M et 1,5 M.

L'hydrolyse alcaline est effectuée au moyen d'une solution alcaline à un pH allant de 13 à 14 pendant une durée allant de 30 minutes à 4 heures, de préférence pendant environ 2 heures.

L'hydrolyse alcaline est effectuée à une température allant de 12 à 40°C, de préférence à température ambiante c'est-à-dire entre 15 et 25 °C.

En outre le ratio masse de tourteau de colza / masse de la solution alcaline est avantageusement compris entre 1/12 et 1/8 et de manière préférée d'environ 1/10.

La dispersion en phase aqueuse et l'hydrolyse alcaline sont avantageusement effectuées sous agitation permettant ainsi une dispersion et une homogénéisation du solide dans le liquide, améliorant ainsi la surface d'échange globale et par conséquent l'hydrolyse.

Après l'étape d'hydrolyse, un acide, de préférence un acide faible, est ajouté pour « neutraliser » la solution c'est-à-dire pour ajuster le pH à une valeur allant de 4 à 5 et en conséquence stabiliser l'hydrolysat obtenu. L'acide faible est de préférence choisi parmi l'acide citrique, l'acide phosphorique, l'acide lactique, l'acide oxalique, l'acide acétique, l'acide glycolique, de manière préférée l'acide citrique. Les acides faibles, notamment citrique et phosphorique, permettent aussi de générer un effet tampon recherché dans les compositions cosmétiques.

Après récupération de l'hydrolysat par séparation solide/liquide, c'est-à-dire récupération de la phase liquide, l'hydrolysat est avantageusement purifié par ultrafiltration.

Enfin, une lyophilisation de l'hydrolysat peut également être effectuée afin de disposer dudit hydrolysat sous forme solide.

Le procédé selon l'invention peut également comprendre des étapes de purification et d'essorage classiques dont la mise en œuvre relève des compétences de l'homme du métier.

## Composition

Comme déjà mentionné, la présente invention vise également une composition cosmétique, comprenant l'hydrolysat de tourteau de colza selon la présente invention.

Avantageusement la composition selon la présente invention comprend de 0,001 à 30 % en poids et de préférence de 0,1 à 20 % en poids d'hydrolysat de tourteau de colza par rapport au poids total de la composition.

Avantageusement la composition cosmétique selon la présente invention comprend, dans un milieu cosmétiquement acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

Avantageusement la composition cosmétique selon la présente invention comprend de 0,001 à 20 % en poids d'au moins un agent cosmétique par rapport au poids total de la composition.

Bien entendu, l'homme du métier veillera à choisir ces agents cosmétiques de manière à ne pas altérer les propriétés de la composition selon l'invention.

La composition selon l'invention peut se présenter sous toutes les formes galéniques normalement utilisées dans le domaine cosmétique, et notamment sous forme d'une poudre ou de pastille, en particulier une pastille de dentifrice, d'un syndet en particulier un shampooing solide, d'une solution aqueuse ou hydroalcoolique, éventuellement gélifiée, éventuellement moussante, d'une dispersion du type lotion éventuellement biphasée, d'une émulsion huile-dans-eau ou eau-dans-huile ou multiple (E/H/E ou H/E/H par exemple), d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non ionique; de gel aqueux ou huileux. Ces compositions sont préparées selon les méthodes usuelles.

La composition selon l'invention peut constituer une composition de soin de la peau, et notamment une crème de nettoyage, de protection, de traitement ou de soin pour la bouche, le visage, pour les mains, pour les pieds, ou pour le corps, en particulier la composition peut être une crème de jour, crème de nuit, crème démaquillante, crème de fond de teint, crème antisolaire ; un fond de teint fluide, un lait de démaquillage, un lait corporel de protection ou de soin, un lait anti-solaire; une lotion, gel ou mousse pour le soin de la peau, comme une lotion de nettoyage.

La composition cosmétique selon la présente invention peut être utilisée en application topique sur la peau à titre d'agent blanchissant et/ou éclaircissant de la peau et/ou dépigmentant de la peau, ainsi qu'à titre d'agent anti-âge de la peau.

Il a été observé que la composition cosmétique selon la présente invention permet de réduire la taille, atténuer voire supprimer les taches brunes de pigmentation, ou encore prévenir, réduire et/ou traiter une altération du teint de la peau. Plus particulièrement, ladite utilisation permet de réduire, atténuer, supprimer les défauts de pigmentation de la peau tels que le mélasma ou masque de grossesse, les lentigos notamment les lentigos solaires, les hyperpigmentations.

Sans vouloir être lié à une quelconque théorie, les inventeurs ont montré que la composition cosmétique selon la présente invention est un inhibiteur de la synthèse de la mélanine dans les mélanocytes humains normaux qui sont les cellules pigmentaires situées dans l'épiderme.

Les inventeurs ont aussi montré que la composition cosmétique selon la présente invention permet une augmentation de l'activité télomérase des kératinocytes humains. La composition cosmétique selon l'invention présente par conséquent des propriétés anti-âge et permet de prévenir et/ou traiter les signes du vieillissement cutané en particulier du photo-vieillissement.

La composition selon l'invention est avantageusement destinée à être appliquée sur la peau.

## Utilisations

La présente invention vise aussi l'utilisation cosmétique, non-thérapeutique d'un hydrolysat selon l'invention ou d'au moins une composition cosmétique selon la présente invention en tant qu'actif pour blanchir et/ou éclaircir et/ou dépigmenter la peau, notamment pour réduire la taille des taches brunes de pigmentation, atténuer voire supprimer les taches brunes de pigmentation, ou encore prévenir, réduire et/ou traiter une altération du teint de la peau.

Plus particulièrement, ladite utilisation permet de réduire, atténuer, supprimer les défauts de pigmentation de la peau.

La présente invention vise encore l'utilisation cosmétique, non-thérapeutique d'un hydrolysat selon l'invention ou d'au moins une composition cosmétique selon la présente invention en tant qu'actif pour prévenir et/ou diminuer les signes de vieillissement cutané, en particulier la perte d'élasticité de la peau, l'altération de la fonction barrière de la peau, l'apparition de rides et/ou de ridules.

La présente invention concerne aussi un procédé de traitement cosmétique, non thérapeutique de la peau, comprenant au moins une étape d'application sur la peau d'un hydrolysat selon l'invention ou d'une composition cosmétique selon l'invention.

Plus particulièrement, l'invention a également pour objet un procédé de traitement cosmétique, non thérapeutique de dépigmentation, d'éclaircissement et/ou de blanchiment de la peau, comprenant au moins une étape d'application sur la peau, d'un hydrolysat selon l'invention ou d'une composition cosmétique selon l'invention.

L'invention a également pour objet un procédé de traitement cosmétique, non thérapeutique et/ou prévention des signes de vieillissement cutané, en particulier la perte d'élasticité de la peau, l'altération de la fonction barrière de la peau, l'apparition de rides et/ou de ridules comprenant au moins une étape d'application sur la peau d'un hydrolysat selon l'invention ou d'une composition cosmétique selon l'invention.

Les exemples qui suivent visent à illustrer l'invention sans en limiter la portée.

## Exemples

### Exemple 1- Extrait de tourteau de colza hydrolysé

### I. Préparation de l'hydrolysat

Une solution alcaline est préparée en mélangeant 80 g d'hydroxyde de sodium 1M et 1920 g d'eau dans un réacteur/hydrolyseur de 3 litres équipé d'un mélangeur à hélice 4 lames (Agitateur IKA RW20).

On introduit dans le réacteur 200 g de tourteau de colza commercialisé par Centre Ouest Céréales préalablement broyé avec une broyeur à couteaux (SM100 RETSCH).

Le mélange est maintenu sous agitation pendant 2 heures à température ambiante.

A l'issue de ces 2 heures, 170 g d'acide citrique monohydraté sont ajoutés et l'agitation est maintenue jusqu'à solubilisation complète de l'acide. Le pH est mesuré, il est égal à 4,68.

Puis 10 g de benzoate de sodium sont ajoutés, à titre de conservateur, et l'agitation est maintenue jusqu'à solubilisation complète du benzoate de sodium. Le pH est mesuré, il est égal à 4,71. Après décantation, le mélange a été filtré sur filtre plissé.

On obtient 1397g de filtrat. L'hydrolysat obtenu comprend un taux de matières sèches de 12.5% en poids par rapport au poids total de l'hydrolysat.

Dans le présent texte, le calcul du taux d'humidité et du taux de matières sèches est déterminé selon le protocole suivant.

On pèse environ 0.3g d'un échantillon du produit à tester dans une coupelle en verre, la masse exacte est nommée Mav, puis on le place dans une étuve pendant 1 heure à 100°C. On pèse à nouveau l'échantillon, la masse exacte est nommée Map.

Le taux d'humidité TH correspond à :

$$TH = (Mav - Map)/ Mav *100$$

On effectue la mesure sur 2 échantillons et on retient la moyenne.

Le taux de matières sèches MS correspond à :

$$MS = 100\text{-}TH.$$

### II. Détermination de la composition de l'hydrolysat

#### A. Analyse par chromatographique gazeuse par détection à ionisation de flamme

La composition en composés volatils du filtrat est analysée par chromatographique gazeuse par détection à ionisation de flamme (GC/FID).

Selon cette méthode, l'échantillon est préparé selon une méthode de micro-extraction en phase solide dite SPME qui utilise commme support solide de piégeage la fibre CAR/PDMS (Carboxen®/Polydimethylsiloxane fibers). Le piégeage a lieu pendant 30 minutes à 90°C.

Afin de pouvoir effectuer une analyse quantitative des composés volatils, 0,4% poids d'acide benzoique, un conservateur, a été introduit dans l'hydrolysat à titre de marqueur.

*Analyse et résultats*

[Tableau 1]

| Tr | N° CAS | Composés | % Fid |
|---|---|---|---|
| 5.00 | 67-64-1 | Acetone | 0.138 |
| 6.11 | 75-15-0 | Carbon disulfide | 0.248 |
| 10.22 | 123-73-9 | 2-Butenal, (E)- | 0.028 |
| 10.70 | 64-19-7 | Acide Acetique | 0.138 |
| 10.90 | 71-43-2 | *Benzene* | *0.216* |
| 15.09 | 3102-33-8 | 3-Penten-2-one, (E)- | 0.044 |
| 15.74 | 1615-70-9 | ***2,4-Pentadienenitrile*** | ***0.348*** |
| 16.45 | 4786-19-0 | ***Methallyl Cyanide*** | ***1.990*** |
| 17.25 | 1615-70-9 | ***2,4-Pentadienenitrile isomère*** | ***0.384*** |
| 17.82 | 4403-61-6 | ***2-Methyl-2-butenenitrile*** | ***0.046*** |
| 18.77 | 66-25-1 | Hexanal | 0.121 |
| 20.95 | 98-01-1 | *Furfural* | *0.503* |
| 22.63 | 5048-19-1 | 5-Cyano-1-pentene | 2.512 |
| 24.43 | 110-43-0 | 2-Heptanone | 0.092 |
| 24.92 | 1516-01-4 | ***2,4-Hexadienenitrile*** | ***0.047*** |
| 25.86 | 123-32-0 | ***2,5-Diméthyl-Pyrazine*** | ***0.562*** |
| 26.84 | 1516-01-4 | ***2,4-Hexadienenitrile isomère*** | ***0.056*** |
| 28.49 | 18829-55-5 | Heptenal<2E-> | 0.018 |
| 28.99 | 100-52-7 | *Benzaldéhyde* | *2.162* |
| 29.52 | 142-62-1 | Acide hexanoique | 0.907 |
| 29.81 | 108-95-2 | *Phenol* | 1.163 |
| 31.08 | 13925-03-6 | ***Pyrazine, 2-ethyl-6-methyl-*** | ***0.089*** |
| 31.25 | 13360-64-0 | ***Pyrazine, 2-ethyl-5-methyl-*** | ***0.187*** |
| 31.36 | 14667-55-1 | ***Pyrazine, trimethyl-*** | ***0.148*** |
| 32.80 | 527-84-4 | *Ortho-Cymène* | *0.115* |
| 33.10 | 138-86-3 | *Limonène* | *0.033* |
| 33.27 | 100-51-6 | *Alcool Benzylique* | *0.221* |
| 33.83 | 122-78-1 | *Benzeneacetaldehyde* | *0.022* |
| 33.94 | 90-02-8 | Salicylaldéhyde | 0.033 |
| 34.80 | 111-14-8 | Acide Heptanoic | 0.541 |
| 35.16 | 98-86-2 | *Acetophenone* | *0.286* |
| 35.66 | 13360-65-1 | ***Pyrazine, 3-ethyl-2,5-dimethyl-*** | ***0.346*** |
| 35.96 | - | ***Pyrazine, 3-ethyl-2,5-dimethyl Isomère*** | ***0.030*** |
| 36.11 | - | ***Pyrazine, 3-ethyl-2,5-dimethyl Isomère*** | ***0.037*** |
| 36.43 | 1195-32-0 | Para-Cyménène | 0.076 |

(suite)

| Tr | N° CAS | Composés | % Fid |
|---|---|---|---|
| 36.71 | 93-58-3 | Benzoate de Méthyle | 0.098 |
| 36.87 | 124-19-6 | n-Nonanal | 0.146 |
| 38.83 | 20893-30-5 | *2-Thiopheneacetonitrile* | *0.043* |
| 38.97 | 140-29-4 | *Benzeneacetonitrile* | *0.094* |
| 41.62 | 65-85-0 | Acide Benzoique | 69.887 |
| 42.17 | 59121-25-4 | *Pentanenitrile, 5-(methylthio)-* | *0.303* |
| 44.25 | 645-59-0 | *3-Phényl-Propionitrile* | *2.988* |
| 45.13 | 112-05-0 | Acide Nonanoique | 2.871 |
| 46.98 | 120-72-9 | *Indole* | *0.482* |
| 47.83 | 7786-61-0 | *2-Methoxy-4-vinylphenol* | 2.565 |
| 49.41 | 334-48-5 | Acide n-Decanoic | 0.222 |
| 50.01 | 104-61-0 | gamma-Nonalactone | 0.203 |
| 51.29 | 92-52-4 | *Biphényle* | *0.045* |
| 56.29 | 96-76-4 | *2,4-Di-tert-butylphenol* | *0.211* |
| 57.89 | 143-07-7 | Acide Dodécanoïque | 0.134 |
| 58.72 | 28343-22-8 | *Phénol, 4-ethenyl-2,6-dimethoxy-* | *0.848* |
| 62.92 | 141-04-8 | Acide hexanedioique, bis(2- methylpropyl) ester | 0.548 |
| Total | | | 95.573 |

Tr correspond au temps de rétention du composé volatil.

D'après le tableau 1, la phase volatile correspond à 0,2% de l'hydrolysat et elle comprend 8,2% de composés azotés (indiqués en gras et italique dans le tableau 1) soit 0,066% de l'hydrolysat. Elle comprend aussi 8,6% de composés aromatiques non azotés (indiqués en italique, non gras dans le tableau 1) soit 0,069% de l'hydrolysat, lesdits composés aromatiques entrant notamment dans la composition d'huiles essentielles.

B. Analyse de la teneur en protéines de l'extrait hydrolysé par la méthode de Lowry

Les quantités de protéines totales présentes ont été déterminées par dosage colorimétrique selon la méthode de Lowry. Pour ce dosage colorimétrique, une courbe étalon de la densité optique, mesurée à 750 nm, en fonction de la quantité de protéines a été réalisée. La protéine de référence utilisée dans cette expérience est la sérum-albumine bovine (BSA).

Puis la détermination de la quantité totale en protéines dans chaque échantillon d'hydrolysat à tester a été obtenue en préparant des échantillons à tester selon le même protocole que celui utilisé pour la gamme étalon puis en reportant les valeurs de DO (densité optique) obtenues pour les échantillons à tester sur la courbe étalon.

*Préparation de la gamme étalon*

Les ingrédients pour préparer la gamme étalon étaient les suivants :

**BSA** : Sérum albumine bovine commercialisé par Sigma Aldrich ® ;
**SDS** : dodécyl sulfate de sodium 20% commercialisé par Biosolve ® ;
**Eau** : WATER PLUS commercialisée par Carlo Erba ® ;
**Na$_2$CO$_3$, NaOH 1M, CuSO4, Tartrate Na/K** et **réactif de Folin-Ciocalteu** commercialisés par Carlo Erba ® ;
**DMSO** commercialisé par Sigma Aldrich ®.

Les solutions suivantes ont été préparées :

**SDS à 0,1 %** : dilution de 1 mL de la solution mère à 20% (m/v) dans 200mL (volume final) d'eau WATER PLUS ;

**BSA à 2 mg/mL** : solubilisation de 2 mg de BSA dans 1mL d'eau WATER PLUS ;

**BSA à 0,2 mg/ml** : dilution de 100 $\mu$L de la solution de BSA à 2 mg/mL dans 1mL (volume final) d'eau WATER PLUS ;

**NaOH 0,1 M** : dilution de 5ML de NaOH 1M dans 50mL (volume final) d'eau WATER PLUS

**Réactif A : Na$_2$CO$_3$ 20g/L, NaOH 0.1M** : Solubilisation d'1g de Na$_2$CO$_3$ dans 50mL de NaOH 0.1M ;

**Réactif B : CuSO$_4$ 5g/l, Tartrate Na/K 10g/L** : Solubilisation de 25mg de CuSO4 et 50mg de Tartrate Na/K dans 5ml d'eau pure de type eau distillée. Le mélange est maintenu à l'obscurité ;

**Réactif C** : 1 ml du Réactif B est ajouté aux 50mL de réactif A. Le mélange est maintenu à l'obscurité ;

**Réactif D (Réactif de Folin-Ciocalteu dilué)** : Dilution de 5ml de réactif de Folin-Ciocalteu commercial dans 5ml d'eau pure de type eau distillée. Le mélange est maintenu à l'obscurité.

La gamme étalon a été réalisée en trois exemplaires, les compositions formant la gamme étalon sont présentées au tableau 2.

[Tableau 2]

| N° de tube | 1 | 2 | 3 | 4 | 5 | 6 | 7 |
|---|---|---|---|---|---|---|---|
| Qantité de BSA ($\mu$g) | 0 | 2,5 | 5 | 10 | 15 | 20 | 25 |
| SDS 0,1% ($\mu$L) | 150 | 137,5 | 125 | 100 | 75 | 50 | 25 |
| Réactif C ($\mu$L) | 750 | | | | | | |
| Réactif D ($\mu$L) | 75 | | | | | | |

*Protocole*

Les quantités de BSA, SDS et réactif C indiquées dans le tableau ci-dessus ont été introduites dans les tubes 1 à 7 puis les tubes ont été mélangés par retournement. Après une pause de 10 minutes dans l'obscurité, 75 $\mu$L de réactif D ont été ajoutés dans chaque tube. Puis les tubes ont été mélangés par retournement. Après une pause de 30 minutes dans l'obscurité, les mesures d'absorbance à 750 nm ont été réalisées.

Les mesures de densité optique à 750 nm ont été effectuées au moyen d'un spectrophotomètre « Genesys 150 » de la société ThermoFisher Scientific.

Les mesures de densité optique à 750 nm ont permis de définir une courbe d'équation : ***y = 0,0164x avec R$^2$ = 0,9877.***

Puis les échantillons à tester ont été préparés selon le protocole suivant : les échantillons sont dilués dans le DMSO selon des dilutions d'un facteur allant de 10 à 200. Puis du SDS à 0,1% a été ajouté pour obtenir un volume total de 150 $\mu$L dans chacun des puits d'essai. Les tubes ont été mélangés par retournement, après une pause de 10 minutes dans l'obscurité, 75 $\mu$L de réactif D ont été ajoutés dans chaque tube. Puis les tubes ont à nouveau été mélangés par retournement, après une pause de 30 minutes dans l'obscurité, des mesures d'absorbance à 750 nm sont réalisées.

Dans le tableau 3 sont présentés les résultats obtenus pour une dilution des échantillons égale à 100. A partir de la courbe étalon, la concentration en protéines de chaque échantillon a été déterminée.

[Tableau 3]

| Volume testé | DO 750 nm | Quantité de protéines ($\mu$g) | Concentration en protéines (dilué) (mg/mL) | Concentration en protéines (mg/mL) |
|---|---|---|---|---|
| 75 | 0,196 | 11,95 | 0,159 | 15,93 |
| 50 | 0,13 | 7,93 | 0,159 | 15,85 |
| 75 | 0,187 | 11,40 | 0,152 | 15,20 |
| 100 | 0,251 | 15,30 | 0,153 | 15,30 |
| 50 | 0,127 | 7,74 | 0,155 | 15,49 |
| 75 | 0,185 | 11,28 | 0,150 | 15,04 |
| 100 | 0,259 | 15,79 | 0,158 | 15,79 |

A partir du tableau 3, on a déduit que l'extrait hydrolysé présentait une teneur en protéines de 15,52 $\pm$ 0,35 mg/mL soit environ 1,55 %. Ce résultat confirme que l'extrait hydrolysé comprend bien des protéines hydrolysées.

C. Détermination du profil de poids moléculaires des protéines

La détermination des poids moléculaires des protéines a été réalisée par électroporèse SDS-Page et coloration au nitrate d'argent.

L'électrophorèse SDS-PAGE en conditions dénaturantes permet la migration des protéines dans un champ électrique uniquement en fonction de leur poids moléculaire. Un gel de polyacrylamide a été coulé puis recouvert d'un tampon de migration qui permet la migration des protéines. Puis les échantillons, ainsi qu'un marqueur de taille, ont été déposés dans les puits du gel puis ont migré à 40V pendant 1 heure puis pendant 2 heures à 100V.

*Préparation des gels d'acrylamide*

2 gels SDS-PAGE de 10 puits, d'épaisseur 1,5mm contenant un gel de séparation (15% acrylamide) et de concentration (4,5% acrylamide) sont coulés.

*Préparation des échantillons*

A 80$\mu$L de l'hydrolysat obtenu au paragraphe I ci-dessus (dilué au 1/10ème dans $H_2O$ distillée) est ajouté 20$\mu$L de tampon de charge 5X (Tris 250mM pH 6,8 ; 10% SDS 10% (m/v) ; Bromophénol blue 0,5% (m/v) ; Glycérol 50% (v/v) ; $\beta$-mercaptoéthanol 15% (v/v))

Les dépôts ont été effectués dans les puits numérotés 2, 4, 6 et 8 comme suit :

- puits 2 : MW = marqueur de taille (BlueStar Plus prestained Protein marker, Nippon Genetics) (3$\mu$L) ;
- puits 4 : E1 (40$\mu$L) ;
- puits 6 : E2 (40$\mu$L) ;
- puits 8 : E3 (40$\mu$L).

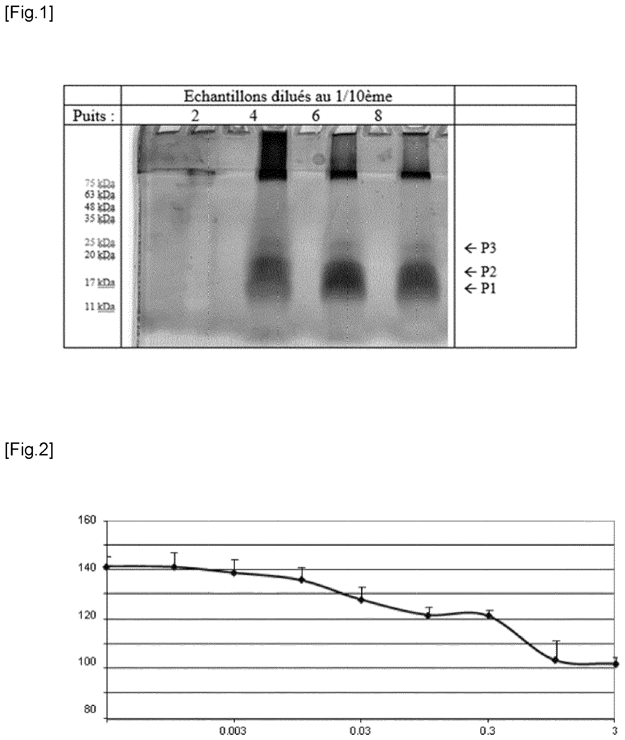

[Fig.1] présente les profils électophorétiques, en révélation au nitrate d'argent, des 3 échantillons d'hydrolysat selon l'invention testés, elle traduit la présence de 3 bandes protéiques nommées P1, P2 et P3 :

- P1 de poids moléculaire apparent d'environ 17kDa ;
- P2 de poids moléculaire apparent comprise entre 17 et 20kDa et
- P3 de poids moléculaire apparent d'environ 25kDa.

La totalité des protéines présente un poids moléculaire inférieur à 30 kDa.

Outre les composés volatils et les protéines, l'hydrolysat est composé de sels alcalins, de tampon citrate et de conservateur.

## Exemple 2 - Cytotoxicité

Pour effectuer les différentes mesures, des compositions de l'hydrolysat de tourteau de colza à différentes concentrations ont été préparées en diluant l'hydrolysat préparé à l'Exemple 1.I ci-dessus directement dans le milieu d'incubation à la concentration visée.

Les cytotoxicités de plusieurs compositions de l'hydrolysat de tourteau de colza à des concentrations différentes ont été évaluées sur des mélanocytes normaux d'épiderme humain cultivés en culture monocouche.

Avant de procéder aux mesures, les compositions d'hydrolysat de tourteau de colza ont été conservées à température ambiante, à l'abri de la lumière.

Les mélanocytes normaux d'épiderme humain ont été cultivés en culture monocouche jusqu'à confluence. Puis les mélanocytes ont été incubés pendant 72 heures soit en l'absence de l'extrait à tester (contrôle) soit en présence des différentes compositions de l'extrait à tester.

La viabilité des mélanocytes a été déterminée par une méthode spectro-colométrique basée sur des mesures d'activité phosphatase alcaline.

Le principe de cette mesure repose sur la transformation du substrat p-nitrophényl phosphate en p-nitrophénol par la phosphatase alcaline intracellulaire des cellules viables, selon la méthode décrite dans « An acid phosphatase assay for quantifying the growth of adherent and nonadherent cells. » Yang T., Sinai P., Kain S. Anal. Biochem., 24, 103-108 (1996).

Dans le tableau 4, les résultats obtenus sont exprimés en pourcentage de cellules viables par rapport au contrôle, la moyenne ainsi que la déviation standard (DS) sont également présentées.

[Fig.2] représente le pourcentage de cellules (mélanocytes) viables par rapport au contrôle en fonction de la concentration de la composition d'hydrolysat de tourteau de colza (vol/vol).

[Tableau 4]

|  | Contrôle | % (v/v) d'hydrolysat de tourteau de colza | | | |
|---|---|---|---|---|---|
|  |  | 0,0003% | 0,001 % | 0,003% | 0,01% |
| % de cellules viables | 99.2 | 126.8 | 126.0 | 123.3 | 116.9 |
|  | 108.6 | 128.4 | 131.1 | 124.3 | 118.7 |
|  | 92.2 | 113.9 | 110.2 | 105.5 | 99.5 |
| **Moyenne** | **100.0** | **123.0** | **122.5** | **117.7** | **111.7** |
| D.S. | 8.2 | 8.0 | 10.9 | 10.6 | 10.6 |
|  | % (v/v) d'hydrolysat de tourteau de colza | | | | |
| % de cellules viables | 0,03% | 0,1% | 0,3% | 1 | 3 |
|  | 102.7 | 89.2 | 88.2 | 39.2 | 40.3 |
|  | 100.8 | 83.8 | 79.0 | 36.2 | 40.0 |
|  | 84.8 | 77.7 | 81.6 | 65.3 | 49.9 |
| **Moyenne** | **96.1** | **83.5** | **82.9** | **46.9** | **43.4** |
| D.S. | 9.8 | 5.7 | 4.8 | 16.0 | 5.6 |

Il ressort de cette étude que l'extrait de tourteau de colza hydrolysé présente un effet cytotoxique sur les mélanocytes normaux d'épiderme humain détectable à partir de la concentration à 0,3% v/v.

*Sur kératinocytes*

Les kératinocytes normaux d'épiderme humain d'un donneur de 66 ans ont été cultivés en culture monocouche jusqu'à confluence. Puis les kératinocytes ont été incubés pendant 48 heures soit en l'absence de l'extrait à tester (contrôle) soit en présence des différentes compositions de l'extrait à tester.

La viabilité des kératinocytes a été déterminée par la méthode spectro-colométrique basée sur des mesures d'activité phosphatase alcaline de Yang *et al* décrite pour les mélanocytes ci-dessus.

Dans le tableau 5, les résultats obtenus sont exprimés en pourcentage de cellules viables par rapport au contrôle, la moyenne ainsi que la déviation standard (DS) sont également présentées.

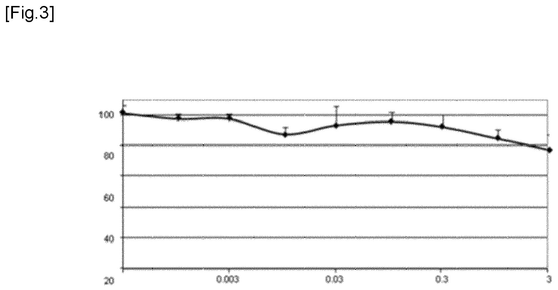

[Fig.3] représente le pourcentage de cellules viables par rapport au contrôle en fonction de la concentration de la composition d'extrait hydrolysé de tourteau de colza (vol/vol).

[Tableau 5]

|  | Contrôle | % (v/v) d'hydrolysat de tourteau de colza | | | |
|---|---|---|---|---|---|
|  |  | 0,0003% | 0,001 % | 0,003% | 0,01% |
| % de cellules viables | 98.5 | 107.0 | 99.4 | 101.5 | 81.6 |
|  | 102.0 | 97.5 | 99.1 | 95.5 | 89.9 |
|  | 99.5 | 98.9 | 93.8 | 95.5 | 89.6 |
| **Moyenne** | **100.0** | **101.1** | **97.4** | **97.5** | **87.1** |
| D.S. | 1.8 | 5.1 | 3.1 | 3.4 | 4.7 |
|  | % (v/v) d'hydrolysat de tourteau de colza | | | | |

(suite)

| | Contrôle | % (v/v) d'hydrolysat de tourteau de colza | | | |
|---|---|---|---|---|---|
| | | 0,0003% | 0,001 % | 0,003% | 0,01% |
| % de cellules viables | 0,03% | 0,1% | 0,3% | 1 | 3 |
| | 102.2 | 102.2 | 101.2 | 90.7 | 66.9 |
| | 98.2 | 92.1 | 88.3 | 81.9 | 80.1 |
| | 78.3 | 92.3 | 86.8 | 80.4 | 84.8 |
| **Moyenne** | **92.9** | **95.5** | **92.1** | **84.3** | **77.2** |
| D.S. | 12.8 | 5.8 | 7.9 | 5.6 | 9.3 |

Il ressort de cette étude que l'extrait hydrolysé de tourteau de présente un effet cytotoxique sur les kératinocytes normaux d'épiderme humain détectable à partir de la concentration à 3% v/v. En outre des modifications de la morphologie des cellules, reflétant un stress, ont été observées à partir de la concentration à 3% v/v.

Cette différence avec les résultats sur mélanocytes est conforme car les kératinocytes appartiennent aux couches supérieures et protectrices de l'épiderme, et sont donc naturellement plus robustes que les mélanocytes appartenant à la couche basale de l'épiderme sous protection des kératinocytes.

Il ressort de ces études de cytotoxicité *in vitro* que des compositions cosmétiques comprenant jusqu'à 30 % en poids d'hydrolysat selon l'invention peuvent être appliquées sur la peau sans risque.

## III Mesures des activités cosmétiques

### 1. Activité dépigmentante

### Mesure de la réduction de production de la mélanine

Les mesures de l'activité dépigmentante (réduction de la production de mélanine) de plusieurs compositions de l'extrait de tourteau de colza hydrolysé présentant des concentrations différentes ont été effectuée par dosage des mélanocytes humains normaux *in vitro*.

Les compositions de concentrations 0,03% (v/v), 0,1% (v/v), et 0,3% (v/v) ont été préparées par dilution dans le milieu d'incubation.

Les cultures des mélanocytes normaux d'épiderme humain ont été cultivés en culture monocouche jusqu'à confluence. Puis les mélanocytes ont été incubés pendant 72 heures soit en l'absence de l'extrait à tester (contrôle) soit en présence des différentes compositions de l'extrait à tester. Le produit de référence pour l'inhibition de la synthèse de mélanine était une solution d'acide kojique à 250 $\mu$M.

Les quantités de mélanine produite par les mélanocytes ont été déterminées par une méthode spectrophotométrique c'est-à-dire une mesure de densité optique à 405 nm effectuée au moyen d'un spectrophotomètre « Genesys 150 » de la société ThermoFisher Scientific.

Le tableau 6 présente la quanité de mélanine ($\mu$g) produite par mg de protéines totales dans le lysat cellulaire monocouche.

Le niveau de signification entre le "contrôle" et la « référence » a été évalué à l'aide d'un test t de Student (* : $p < 0,05$).

[Tableau 6]

| | Contrôle | Concentration de l'extrait hydrolysé (%v/v) | | | Référence acide kojique à 250 $\mu$M. |
|---|---|---|---|---|---|
| | | 0,03% | 0,1% | 0,3% | |
| $\mu$g mélanine/mg de protéines | 64.7 | 36.6 | 43.7 | 65.8 | 45.1 |
| | 64.5 | 44.1 | 60.8 | 53.7 | 30.3 |
| | 75.8 | 45.1 | 54.6 | 55.2 | 32.0 |
| Moyenne | 68.3 | 42.0** | 53.0 | 58.2 | 35.8** |
| D.S. | 6.5 | 4.6 | 8.7 | 6.6 | 8.1 |

(suite)

| | Contrôle | Concentration de l'extrait hydrolysé (%v/v) | | | Référence acide kojique à 250 µM. |
|---|---|---|---|---|---|
| | | 0,03% | 0,1% | 0,3% | |
| % du contrôle | 100 | 61,4% (p<0,01) | 77,6% (p>0,05) | 85,2% (p>0,05) | 52.4 (p<0,01) |

On observe en particulier que l'extrait de tourteau de colza hydrolysé à la concentration de 0,03% inhibe de manière significative la production de mélanine de plus de 38,6% (61,4% du contrôle). Les autres échantillons présentant également une inhibition de la production de mélanine indiquent et confirment la tendance dépigmentante de l'extrait selon l'invention. Le produit de référence inhibe de manière significative la production de mélanine de 47,6% ce résultat était attendu et valide ce test d'inhibition.

Les compositions selon la présente invention présentent donc un effet éclaircissant, blanchissant, dépimentant.

**2.Activité Anti-âge**

**A/ Mesure de l'augmentation de l'activité télomérase**

Les mesures de l'activité télomérase de plusieurs compositions de l'extrait de tourteau de colza hydrolysé à des concentrations différentes ont été effectuées sur des kératinocytes humains adultes normaux à un faible degré de passage cultivés en monocouche. Par « faible degré de passage », on entend des cellules fraîches qui n'ont pas subi (voire très peu) de cycles de mitoses.

Les compositions de concentrations 0,1% (v/v), 0,5% (v/v), et 2% (v/v) ont été préparées par dilution dans le milieu d'incubation.

Les cultures des kératinocytes normaux d'épiderme humain ont été cultivés en culture monocouche jusqu'à 75 % de confluence.

Puis les kératinocytes ont été incubés pendant 24 heures soit en l'absence de l'extrait à tester (contrôle) soit en présence des différentes compositions de l'extrait à tester. Le témoin utilisé en tant que référence pour l'induction de l'activité télomérase était une solution de depsipeptide FK228 à 100 ng/mL.

*Mesures des protéines*

À la fin de la période d'incubation, les protéines totales des cellules ont été extraites des cellules et mesurées en utilisant la méthode spectrocolorimétrique dite de « Bradford ». Cette mesure a permis de déterminer le volume exact d'extrait à utiliser lors de la mesure de l'activité télomérase, afin de maintenir la même quantité de protéines (contenant de la télomérase) pour toutes les conditions testées au niveau de l'étape de « PCR ».

*Mesures de l'activité télomérase*

À la fin de la période d'incubation, la télomérase est extraite des cellules et son activité est déterminée à l'aide d'un kit spécifique et sensible. Le principe du kit de mesure de l'activité de la télomérase couple une étape PCR (dans laquelle la télomérase effectue l'élongation) avec une étape ELISA permettant la détermination semi-quantitative des quantités de produits d'élongation de la télomérase. La quantité d'anticorps qui se lient spécifiquement sur les télomères néoformés est corrélée à la longueur (plus le télomère est long, plus il y a de place pour lier les anticorps) et comme les anticorps portent le signal mesuré, l'intensité du signal est corrélée à la longueur des télomères néoformés.

Le tableau 7 présente les résultats de l'activité télomérase en valeurs par rapport à 1 (100%) qui correspond à la taille normale de télomère néoformé obtenu avec les cellules normales dans du milieu de culture (sans l'hydrolysat selon l'invention), sont également présentées les moyenne et déviation standard SD.

Le niveau de signification entre le "véhicule", c'est-à-dire le DMSO et le "produit de référence" a été évalué à l'aide d'un test t de Student (* : $p<0,05$).

Le niveau de signification entre le "contrôle" et "composé testé" a été évalué indépendamment pour chaque composition par une analyse de variance à un facteur (ANOVA à sens unique) suivie d'un test de Holm-Sidak (* : $p<0,05$).

[Tableau 7]

| | contrôle | Concentration de l'extrait hydrolysé (%v/v) | | | Témoin FK228 | Véhicule DMSO |
|---|---|---|---|---|---|---|
| | | 0,1% | 0,5% | 2% | | |
| Activité télomérase | 0.554 | 0.643 | 0.652 | 0.640 | 0.796 | 0.519 |
| | 0.550 | 0.639 | 0.628 | 0.685 | 0.757 | 0.526 |
| | 0.523 | 0.601 | 0.635 | 0.621 | 0.736 | 0.579 |
| **Moyenne** | **0.542** | **0.628\*\*** | **0.638\*\*** | **0.649\*\*** | **0.763\*\*** | **0.541** |
| D.S. | 0.017 | 0.023 | 0.012 | 0.033 | 0.030 | 0.033 |
| % du contrôle | 100.0 | 115.8 | 117.7 | 119.6 | 140.7 | 99.8 |
| % du véhicule | | | | | 141.0 | |
| D.S. | 3.1 | 4.3 | 2.3 | 6.0 | 5.5 | 6.0 |

On observe que l'extrait de tourteau de colza hydrolysé selon l'invention a la capacité de stimuler de manière significative l'activité télomérase dès la concentration de 0,1%.

L'extrait selon la présente invention présente un effet vis-à-vis du traitement des signes de vieillissement cutané en agissant au cœur du noyau cellulaire des kératinocytes de l'épiderme.

**Exemple 4- Compositions cosmétiques**

Les compositions selon l'invention suivantes ont été réalisées.

4.1 Crème de corps anti-âge

[Tableau 8]

| Composant | g |
|---|---|
| Hydrolysat de tourteau de colza selon l'exemple 1 | 1,0 |
| Urée (UREE PERLES 46% commercialisé par Brenntag ®) | 1,0 |
| Acide lactique (80% M.A.) | 0,2 |
| Acide salicylique | 0,3 |
| Benzoate de sodium | 0,5 |
| Gomme de xanthane (FFPC commercialisé par Jungbunzlauer ®) | 0,2 |
| Glycérine végétale (Palmera G995E commercialisé par Interchimie ®) | 3,5 |
| Bentonite/ gomme de xanthane/stéaroyle de sodium glutamate / acide citrique (Frametime CXG commercialisé par Ephyla) | 3,2 |
| Alcool cétostéarylique 50/50 | 1,0 |
| Beurre de karité | 1,5 |
| Huile de colza | 16,5 |
| Parfum | 0, 5 |
| Eau | 70,7 |

4.2 Gel anti-âge éclat

[Tableau 9]

| Composant | g |
|---|---|
| Hydrolysat de tourteau de colza selon l'exemple 1 | 2,000 |
| Poudre de jus de feuille d'aloe vera (Aloe Vera Gel 1X AG002 commercialisé par Rahn ®) | 0,4635 |
| Gomme d'acaccia du senegal (Fibregum bio commercialisé par Nexira ®) | 1,5000 |
| Gomme de xanthane (FFPC commercialisé par Jungbunzlauer) | 1,3000 |
| Benzoate de sodium | 0,5000 |
| Glycérine végétale (Palmera G995E commercialisé par Interchimie ®) | 4,0000 |
| Eau | 92,2365 |

Ces compositions cosmétiques appliquées quotidiennement sur l'ensemble du corps permettent d'éclaircir la peau, et d'atténuer le vieillissement cutané. La peau est moins terne, plus homogène, moins sèche. Les peaux traitées sont plus lumineuses, plus rayonnantes.

4.3 Shampooing douche éclat

[Tableau 10]

| Composant | g |
|---|---|
| Hydrolysat de tourteau de colza selon l'exemple 1 | 1,000 |
| Bentonite/ gomme de xanthane/stéaroyle de sodium glutamate / acide citrique (Frametime CXG commercialisé par Ephyla) | 4,725 |
| Coco-sulfate de sodium (SULFETAL C90C) | 5,100 |
| Isethionate de cocoyl sodium (ELFAN AT 84) | 4,050 |
| Acide salicylique | 0,300 |
| Benzoate de sodium | 0,495 |
| Gomme de xanthane (FFPC commercialisé par Jungbunzlauer) | 0,105 |
| Pigments (COVAPEARL ANTIQUE SILVER 239) | 0,225 |
| Parfum | 0,500 |
| Eau | 83,500 |

Appliqué quotidiennement sur l'ensemble du corps, ce produit permet l'obtention d'une peau moins sèche, plus lumineuse et rayonnante.

**Revendications**

1.   Hydrolysat de tourteau de colza **caractérisé en ce qu'**il comprend de 0,3 à 3% en poids de protéines de poids moléculaire inférieur à 30 kDa par rapport au poids total de l'hydrolysat et de 0,01 % à 0,5 % en poids de composés volatils par rapport au poids total de l'hydrolysat, dont 0,01 à 0,1% en poids de composés volatils azotés par rapport au poids total de l'hydrolysat, un composé volatil étant un composé dont la pression de vapeur à 20°C (293,15 K) est supérieure ou égale à 0,01 kPa.

2.   Hydrolysat de tourteau de colza selon la revendication 1 dans lequel la phase des composés volatils comprend de 25% à 35% de composés azotés en poids par rapport au poids total de la phase des composés volatils, de 30% à 45% de composés aromatiques non azotés en poids par rapport au poids total de la phase des composés volatils.

**3.** Hydrolysat de tourteau de colza selon la revendication 1 ou 2 **caractérisé en ce qu'**il est obtenu par hydrolyse alcaline.

**4.** Procédé de préparation de l'hydrolysat selon l'une quelconque des revendications précédentes comprenant au moins les étapes suivantes, dans cet ordre :

- fournir des graines de colza ;
- presser les graines de colza et récupérer le tourteau ;
- placer le tourteau de colza en phase aqueuse ;
- soumettre le tourteau à au moins une étape d'hydrolyse au moyen d'une solution alcaline à un pH allant de 13 à 14 pendant une durée allant de 30 minutes à 4 heures,
- ajouter un acide faible de manière à ajuster le pH à une valeur allant de 4 à 5 ;
- effectuer une séparation solide/ liquide,
- récupérer l'hydrolysat obtenu.

**5.** Procédé selon la revendication précédente dans lequel, lors de l'hydrolyse, le ratio masse de tourteau de colza / masse de la solution alcaline est compris entre 1/12 et 1/8 et de manière préférée d'environ 1/10.

**6.** Composition cosmétique ou alimentaire **caractérisée en ce qu'**elle comprend au moins un hydrolysat selon l'une quelconque des revendications 1 ou 2 ou préparé suivant le procédé selon l'une quelconque des revendications 3 ou 4, de préférence en une teneur allant de 0,001 à 30 % en poids et de préférence de 0,1 à 20 % en poids par rapport au poids total de la composition.

**7.** Composition cosmétique selon la revendication précédente comprenant, dans un milieu cosmétiquement acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

**8.** Composition cosmétique selon l'une quelconque des revendications 5 ou 6 **caractérisée en ce qu'**elle est destinée à être appliquée sur la peau.

**9.** Utilisation cosmétique, non thérapeutique, d'un hydrolysat selon l'une quelconque des revendications 1 ou 2 ou préparé suivant le procédé selon l'une quelconque des revendications 3 ou 4 ou d'au moins une composition cosmétique selon l'une quelconque des revendications 5 à 7 en tant qu'actif pour prévenir et/ou diminuer les signes de vieillissement cutané, en particulier la perte d'élasticité de la peau, l'altération de la fonction barrière de la peau, l'apparition de rides et/ou de ridules.

**10.** Utilisation cosmétique, non thérapeutique, d'un hydrolysat selon l'une quelconque des revendications 1 ou 2 ou préparé suivant le procédé selon l'une quelconque des revendications 3 ou 4 ou d'au moins une composition cosmétique selon l'une quelconque des revendications 5 à 7 en tant qu'actif pour blanchir et/ou éclaircir et/ou dépigmenter la peau, notamment pour réduire la taille des taches brunes de pigmentation, atténuer voire supprimer les taches brunes de pigmentation, ou encore prévenir, réduire et/ou traiter une altération du teint de la peau.

**11.** Procédé de traitement cosmétique, non thérapeutique de la peau, comprenant au moins une étape d'application sur la peau, d'un hydrolysat selon l'une quelconque des revendications 1 ou 2 ou préparé suivant le procédé selon l'une quelconque des revendications 3 ou 4 ou d'une composition cosmétique selon l'une quelconque des revendications 5 à 7.

**12.** Procédé de traitement cosmétique, non thérapeutique et/ou de prévention des signes de vieillissement cutané, en particulier la perte d'élasticité de la peau, l'altération de la fonction barrière de la peau, l'apparition de rides et/ou de ridules comprenant au moins une étape d'application sur la peau d'un hydrolysat selon l'une quelconque des revendications 1 ou 2 ou préparé suivant le procédé selon l'une quelconque des revendications 3 ou 4 ou d'une composition cosmétique selon l'une quelconque des revendications 5 à 7 [Revendication 13] Procédé de traitement cosmétique, non thérapeutique de dépigmentation, d'éclaircissement et/ou de blanchiment de la peau comprenant au moins une étape d'application sur la peau d'un hydrolysat selon l'une quelconque des revendications 1 ou 2 ou préparé suivant le procédé selon l'une quelconque des revendications 3 ou 4 ou d'une composition cosmétique selon l'une quelconque des revendications 5 à 7.

[Fig.1]

[Fig.2]

[Fig.3]

Europäisches
Patentamt

European
Patent Office

Office européen
des brevets

**RAPPORT DE RECHERCHE EUROPEENNE**

Numéro de la demande

**EP 22 17 7302**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | CHABANON ET AL: "Hydrolysis of rapeseed protein isolates: Kinetics, characterization and functional properties of hydrolysates", PROCESS BIOCHEMISTRY, ELSEVIER LTD, GB, vol. 42, no. 10, 15 septembre 2007 (2007-09-15), pages 1419-1428, XP022250164, ISSN: 1359-5113, DOI: 10.1016/J.PROCBIO.2007.07.009 * Séction 2.2 * | 1-13 | INV. A61Q19/02 A61Q19/08 A61K8/9789 |
| Y | EP 2 931 382 B1 (HENKEL AG & CO KGAA [DE]) 27 juin 2018 (2018-06-27) * revendications 1-6 * * alinéa [0003] * | 1-13 | |
| Y | US 8 309 144 B2 (DAL FARRA CLAUDE [US]; DOMLOGE NOUHA [FR] ET AL.) 13 novembre 2012 (2012-11-13) * revendications * | 1-13 | |
| Y | CA 2 590 693 A1 (PF MEDICAMENT [FR]; CENTRE NAT RECH SCIENT [FR]) 22 juin 2006 (2006-06-22) * exemple 1 * * page 13, ligne 1 - ligne 3 * | 1-13 | DOMAINES TECHNIQUES RECHERCHES (IPC) A61Q A61K |
| Y | JOHN WOODRUFF: "Skin Lightening – Light and smooth and even", INTERNET CITATION, 21 août 2008 (2008-08-21), XP007910592, Extrait de l'Internet: URL:Skin Lightening – Light and smooth and even – cosmetics business [extrait le 2009-11-20] * Séction "Natural lighteners"; page 3 * | 1-13 | |

-/--

Le présent rapport a été établi pour toutes les revendications

| Lieu de la recherche | Date d'achèvement de la recherche | Examinateur |
|---|---|---|
| Munich | 9 janvier 2023 | Cismaru, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

Europäisches
Patentamt

European
Patent Office

Office européen
des brevets

# RAPPORT DE RECHERCHE EUROPEENNE

Numéro de la demande

**EP 22 17 7302**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | "Vincience(TM) Biofunctionals: Skin Care and Hair Care Applications", INTERNET CITATION, 1 janvier 2015 (2015-01-01), pages 1-19, XP002749061, Extrait de l'Internet: URL:https://www.ulprospector.com/en/na/PersonalCare/Detail/305/585194/Elixiance-biofunctional [extrait le 2015-10-26] * page 2 * ----- | 1-13 | |
| Y | FR 2 925 325 A1 (VINCIENCE SA [FR]) 26 juin 2009 (2009-06-26) * exemple 1 * * revendications * ----- | 1-13 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 9 janvier 2023 | Cismaru, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**EP 4 134 135 A1**

## ANNEXE AU RAPPORT DE RECHERCHE EUROPEENNE
## RELATIF A LA DEMANDE DE BREVET EUROPEEN NO.

EP 22 17 7302

09-01-2023

| Document brevet cité au rapport de recherche | | Date de publication | Membre(s) de la famille de brevet(s) | | Date de publication |
|---|---|---|---|---|---|
| EP 2931382 | B1 | 27-06-2018 | CN | 104837531 A | 12-08-2015 |
| | | | DE | 102012222970 A1 | 12-06-2014 |
| | | | EP | 2931382 A2 | 21-10-2015 |
| | | | WO | 2014090526 A2 | 19-06-2014 |
| US 8309144 | B2 | 13-11-2012 | AUCUN | | |
| CA 2590693 | A1 | 22-06-2006 | AU | 2005315612 A1 | 22-06-2006 |
| | | | BR | PI0518634 A2 | 02-12-2008 |
| | | | CA | 2590693 A1 | 22-06-2006 |
| | | | CN | 101084304 A | 05-12-2007 |
| | | | EP | 1824963 A1 | 29-08-2007 |
| | | | FR | 2879214 A1 | 16-06-2006 |
| | | | JP | 2008522637 A | 03-07-2008 |
| | | | KR | 20070107680 A | 07-11-2007 |
| | | | US | 2009124010 A1 | 14-05-2009 |
| | | | US | 2012088303 A1 | 12-04-2012 |
| | | | WO | 2006064020 A1 | 22-06-2006 |
| FR 2925325 | A1 | 26-06-2009 | AUCUN | | |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **OLIVER H. ; LOWRY, NIRA ; J. ROSEBROUGH ; A LEWIS FARR ; ROSE J. RANDALL.** Protein measurement with the Folin phenol reagent. *J. biol. Chem.,* 1951, vol. 193 (1), 265-275

- **YANG T. ; SINAI P. ; KAIN S.** An acid phosphatase assay for quantifying the growth of adherent and non-adherent cells. *Anal. Biochem.,* 1996, vol. 24, 103-108